# EUROPEAN PATENT APPLICATION

(11) **EP 1 767 939 A1**
(43) Date of publication of application: **28.03.2007**
(21) Application number: 06120554.8
(22) Date of filing: 13.09.2006
(51) Int. Cl.: G01N 33/50, G01N 33/68, C12Q 1/68

(54) **FABP4 as marker for a toxic effect**

(30) Priority: 23.09.2005 EP 05108821
(71) Applicant: F. HOFFMANN-LA ROCHE AG, 4070 Basel (CH)
(72) Inventor: Boess, Franziska, 4125 Riehen (CH); Suter-Dick, Laura, 4103 Bottmingen (CH)

(57) **Abstract**

The present invention relates to FABP4 as marker for determining at least one toxic effect of a compound of interest.

## Description

The gene expression pattern governs cellular development and physiology, and is affected by pathological situations, including disease and the response to a toxic insult. Bearing this in mind, it becomes clear that the study of gene and protein expression in preclinical safety experiments will help toxicologists to better understand the effects of chemical exposure on mammalian physiology. On the one hand, the identification of a certain number of modulated genes and/or proteins after exposure to a toxicant will lead to the identification of novel predictive and more sensitive biomarkers which might replace the ones currently used. The knowledge regarding marker genes for particular mechanisms of toxicity, together with the rapidly growing understanding of the structure of the human genome will form the basis for the identification of new biomarkers. These markers may allow the prediction of toxic liabilities, the differentiation of species-specific responses and the identification of responder and non-responder populations. Gene expression analysis is an extremely powerful tool for the detection of new, specific and sensitive markers for given mechanisms of toxicity (Fielden, M. R., and Zacharewski, T. R. (2001). Challenges and limitations of gene expression profiling in mechanistic and predictive toxicology. Toxicol Sci 60, 6-10). These markers should provide additional endpoints for inclusion into early animal studies, thus minimizing the time, the cost and the number of animals needed to identify the toxic potential of a compound in development.

The present invention is based on a PPARalpha/gamma co-agonist showing unexpected toxicity in mice. The main target organ was the liver, showing dose dependent coagulative necrosis. Also, the compound caused degeneration of heart and skeletal muscle, and brown adipose tissue. Gene expression analysis in the livers revealed effects on lipolysis, including a strong induction of fatty acid binding protein 4 which was not detected in the control.

Fatty acid binding proteins (FABP) are a family of small, highly conserved, cytoplasmic proteins that bind long-chain fatty acids and other hydrophobic ligands. It is thought that FABPs roles include fatty acid uptake, transport and metabolism. FABP4 is usually not expressed in the liver cells but in adipocytes where it increases lipolysis.

The present invention provides FABP4 as biomarker for determining at least one toxic effect of a candidate compound. Preferably, the at least toxic effect is a hepatotoxic effect. In a preferred embodiment, the predicted hepatotoxicity is liver necrosis.

Moreover, the present invention provides the use of FABP4 as biomarker for determining at least one toxic effect of a candidate compound. Preferably, the toxic effect is a hepatotoxic effect. More preferably, the toxic effect is liver necrosis.

The present invention also provides a method for predicting at least one toxic effect of a candidate compound comprising
a) detecting the level of expression of the FABP4 gene in a tissue or cell sample exposed to the compound,
b) comparing the level of expression of gene to its level of expression in a control tissue or cell sample, wherein a differential expression of the gene is indicative of at least one toxic effect.

Preferably, the differential expression of the gene is an increased expression. More preferably, an increased expression of FABP4 gene in the tissue or cell sample exposed to the compound in comparison to the control sample is indicative of at least one toxic effect.A control sample is a sample not exposed to the candidate compound.

The term "tissue or cell sample exposed to the candidate compound" means that the tissue or cell sample or the animal from which the sample is derived is treated with the candidate compound.

A "toxic effect" as used herein refers to a deleterious effect on a living organism, organ system, individal organ, tissue, cell or subcellular unit attributed to the presence of a compound. A toxic effect may be a physiologic or physical manifestation or derangement such as necrosis of cells or organs.

A "candidate compound" as used herein refers to any compound which shall be tested for its toxicity.

The person skilled in the art is familiar with different methods of measuring the level of RNA or protein. The term "level" relates to amount or concentration of an RNA or protein or fragments thereof in an individual or a sample taken from an individual. Measuring a FAPB4 RNA or protein includes also the measuring a fragment or a variant of the RNA or protein.

Methods for measuring the RNA level of FABP4 comprise for example Northern Blotting, quantitation of the bands by densitometry. Preferred are methods comprising, microarray analysis (gene chip), dot blotting or different quantitative PCR methodologies. More preferably, a microarray analysis is used for measuring the level of expression of FABP4 gene.

Methods for measuring the level of FABP4- protein comprise for example precipitation (particularly immunoprecipitation), electrochemiluminescence (electrogenerated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CEMS).

Further methods known in the art (such as gel electrophoresis, 2D gel electrophoresis, SDS polyacrylamid gel electrophoresis (SDS-PAGE), Western Blotting), can be used alone or in combination with labelling or other detection methods.

FABP4 may be used as marker in any mammal such as human rat, mouse, or monkey (human FABP4: SEQ. ID NO: 1, rat FAPB4: SEQ. ID NO: 2, mouse FABP4: SEQ. ID NO: 3). Preferably, FABP4 is used as marker in mouse or rat.

The term "variant" of a RNA in this context relates to nucleotides substantially similar to said RNA. The term "substantially similar" is well understood by the person skilled in the art. In particular, a variant may be an isoform or allele which shows nucleotide exchanges compared to the nucleotide sequence of the most prevalent RNA isoform in the respective population. Preferably, such a substantially similar RNA has a sequence similarity to the most prevalent isoform of the RNA of at least 80%, preferably at least 85%, more preferably at least 90%, most preferably at least 95%. Substantially similar are also degradation products, e.g. nuclease degradation products, which are still recognized by the diagnostic means or by ligands directed against the respective full-length RNA.

The term "variant" of a protein in this context relates to proteins or peptides substantially similar to said protein. The term "substantially similar" is well understood by the person skilled in the art. In particular, a variant may be an isoform or allele which shows amino acid exchanges compared to the amino acid sequence of the most prevalent peptide isoform in the respective population. Preferably, such a substantially similar peptide has a sequence similarity to the most prevalent isoform of the protein or peptide of at least 80%, preferably at least 85%, more preferably at least 90%, most preferably at least 95%. Substantially similar are also degradation products, e.g. proteolytic degradation products, which are still recognized by the diagnostic means or by ligands directed against the respective full-length protein or peptide. The term "variants" is also meant to relate to splice variants.

The term "variant" also relates to a post-translation ally modified protein such as glycosylated protein. A "variant" is also a peptide which has been modified after collection of the sample, for example by covalent or non-covalent attachment of a label, particularly a radioactive or fluorescent label, to the protein.

Having now generally described this invention, the same will become better understood by reference to the specific examples, which are included herein for purpose of illustration only and are not intended to be limiting unless otherwise specified, in connection with the following figures.

### Figures

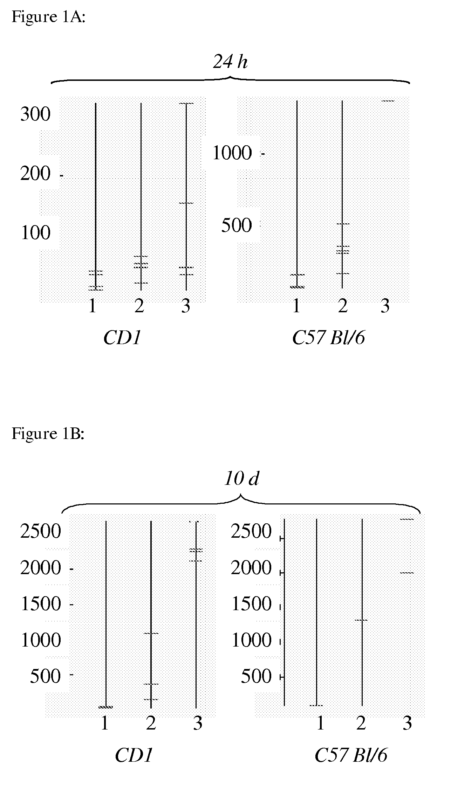

Figure 1 shows a schematic representation of the expression levels of mouse FABP4 (SEQ. ID NO: 3) in livers of CD1 and C57B1/6 mice treated with different doses of a PPARalpha/gamma co-antagonist 3-{1-[2-(2-CHLORO-PHENYL)-5-METHYL-OXAZOL-4-YLMETHYL]-1H-INDOL-5-YL}-2-ETHOXY-PROPIONIC ACID (known to cause liver necrosis after repeated administration to the animals at the tested doses) measured with Affymetrix microarray (MEA 230 plus). 1: Control (0mg/kg), 2: 0.2 mg/kg; 3: 3 mg/kg
A: at 24h after initial treatment.
B: at 10 days after initial treatment (daily administration)

### Examples

Commercially available reagents referred to in the example were used according to manufacturer's instructions unless otherwise indicated.

### Example 1:

### Animals and Doses:

### CD-1 mice (Charles River Laboratories, UK):

- treatment with 3-{1-[2-(2-CHIDRO-PHENYL)-5-METHYL-OXAZOL-4-YLMETHYL]-1H-INDOL-5-YL}-2-ETHOXY-PROPIONIC ACID for 1 day; doses were 0, 0.2 and 3 mg/kg
- treatment with 3-{1-[2-(2-CHLORO-PHENYL)-5-METHYL-OXAZOL-4-YLMETHYL]-1H-INDOL-5-YL}-2-ETHOXY-PROPIONIC ACID for 10 days; doses were 0, 0.2 and 3 mg/kg
- treatment with 3-{1-[2-(2-CHLORO-PHENYL)-5-METHYL-OXAZOL-4-YLMETHYL]-1H-INDOL-5-YL}-2-ETHOXY-PROPIONIC ACID for 14 days; doses were 0, 0.6 and 15 mg/kg

### C57BL6 mice (RCC Ltd, Füllinsdorf):

- treatment with 3-{1-[2-(2-CHLORO-PHENYL)-5-METHYL-OXAZOL-4-YLMETHYL]-1H-INDOL-5-YL}-2-ETHOXY-PROPIONIC ACID for 1 day; doses were 0, 0.2 and 3 mg/kg
- treatment with 3-{1-[2-(2-CHLORO-PHENYL)-5-METHYL-OXAZOL-4-YLMETHYL]-1H-INDOL-5-YL}-2-ETHOXY-PROPIONIC ACID for 10 days; doses were 0, 0.2 and 3 mg/kg

### Wistar rats (RCC Ltd, Füllinsdorf):

- treatment with 3-{1-[2-(2-CHLORO-PHENYL)-5-METHYL-OXAZOL-4-YLMETHYL]-1H-INDOL-5-YL}-2-ETHOXY-PROPIONIC ACID for 14 days; doses were 0, 0.3 and 1.2 mg/kg
- Treatment with 3-{1-[2-(2-CHLORO-PHENYL)-5-METHYL-OXAZOL-4-YLMETHYL]-1H-INDOL-5-YL}-2-ETHOXY-PROPIONIC ACID for 28 days; doses were 0, 0.1, 0.5 and 2.5 mg/kg

### Cynomolgus Monkey (macaca fascicularis):

- treatment with 3-{1-[2-(2-CHLORO-PHENYL)-5-METHYL-OXAZOL-4-YLMETHYL]-1H-INDOL-5-YL}-2-ETHOXY-PROPIONIC ACID for 28 days; doses were 0, 0.015, 0.09 and 0.31 mg/kg
- Treatment with 3-{1-[2-(2-CHLORO-PHENYL)-5-METHYL-OXAZOL-4-YLMETHYL]-1H-INDOL-5-YL}-2-ETHOXY-PROPIONIC ACID for 16 weeks; doses were 0, 0.15, 0.1, 0.3 and 1 mg/kg

3-{1-[2-(2-CHLORO-PHENYL)-5-METHYL-OXAZOL-4-YLMETHYL]-1H-INDOL-5-YL}-2-ETHOXY-PROPIONIC ACID was dissolved in 20% propylene glycol/phosphate buffer, pH8. Treated animals were dosed orally (p.o.).

The expression levels of FABP4 of the mice (SEQ. ID NO: 3) were measured using Affymetrix microarrays (MEA 230 plus, probe sets: 1417023_a_at, 1424155_at, 1425809_at, 1451263_a_at) or Applied Biosystems Assays on Demand (assay ID: Mm00445880_m1). The expression levels of FABP4 of the rat (SEQ. ID NO: 2) were measured using Affymetrix microarrays (RG U34A, probe set: rc_AI169612_at) or Applied Biosystems Assays on Demand (assay ID: Rn00670361_ml). The expression levels of FABP4 of the monkeys were not measured.

### Results:

A dose-dependent severe toxicity at ≥ approximately 50x anticipated human exposure was found in mice with 3-{1-[2-(2-CHLORO-PHENYL)-5-METHYL-OXAZOL-4-YLMETHYL]-1H-INDOL-5-YL}-2-ETHOXY-PROPIONIC ACID (PPAR α,γ-co-agonist). The main target organ was the liver, showing dose dependent coagulative necrosis. Also, the compound caused degeneration of heart and skeletal muscle, and brown adipose tissue. Mortality in mice occurred at ≥0.6 mg/kg/day.

In rat and monkey tissue (liver, heart, skeletal muscle, Brown adipose tissue) such toxicity was not observed at similar exposure levels.

The gene expression in the liver after administration of 3-{1-[2-(2-CHLOROPHENYL)-5-METHYL-OXAZOL-4-YLMETHYL]-1H-INDOL-5-YL}-2-ETHOXY PROPIONIC ACID was compared between rats and mice. The comparison revealed a strong induction of the expression of fatty acid binding protein 4 in mice (see Figure 1). FABP 4 was not induced in rats.

For other compounds eliciting liver toxicity in the rat (indomethacine (see example 2), lipopolysaccharide (LPS), carbon tetrachloride (CCl₄)) an induction of the expression of FABP4 was also found in rat liver tissue.

### Example 2:

### Animals and Doses:

### Wistar rats (RCC Ltd, Füllinsdorf):

- treatment with INDOMETHACIN (1-(4-Chlorobenzoyl)-5-methoxy-2-methyl-3-indoleacetic acid, Sigma 17378), single dose p.o. (orally); doses were 0, 2, 10 and 20 mg/kg
- Treatment with INDOMETHACIN (1-(4-Chlorobenzoyl)-5-methoxy-2-methyl-3-indoleacetic acid, Sigma 17378) for 7 days; doses were 0, 2, and 5 mg/kg/day
INDOMETHACIN was dissolved in corn oil. Treated animals were dosed orally (p.o.).
The expression levels of FABP4 of the rat (SEQ. ID NO: 2) were measured using Affymetrix microarrays (RG U34A, probe sets: rc_AI169612_at) or Applied Biosystems Assays on Demand (assay ID: Rn00670361_ml).

### Results:

Slight histopathological alterations (hepatocellular hypertrophy) was observed in a dose dependent manner in the livers of indomethacin treated. Decreased hepatocellular glycogen deposition in test-item treated animals was observed after single administration of 10 or 20 mg/kg or multiple administration of 5 mg/kg/day for 7 consecutive days. These finding were accompanied by changes of different clinical biochemistry parameters indicative of liver changes especially in the 10 and 20 mg/kg dose groups after single dose indomethacin administration and the 5 mg/kg/day dose group after repeated treatment.

The gene expression in the liver after administration of indomethacin was measured using Affymetrix gene arrays and a strong induction of the expression of fatty acid binding protein 4 (see Table 1) was observed in rats 24 hours after single dose treatment with 10 or 20 mg/kg indomethacin and in the livers of rats treated for 7 consecutive days with 5 mg/kg/day indomethacin. For the treatment groups with the highest induction (20 mg/kg for 24 hours and 5 mg/kg/day for 7 days), this induction of FABP4 mRNA was confirmed by PCR. (see Table 1)

| Treatment | Time of necropsy | Dose (mg/kg) | FABP4 expression relative to time matched vehicle control | |
|---|---|---|---|---|
| | | | Affymetrix result (Fold change) | PCR result (Fold change) |
| vehicle | 6 hours | 0 | 1 | n.d. |
| indomethacin | 6 hours | 2 | 1 | n.d. |
| indomethacin | 6 hours | 10 | 1 | n.d. |
| indomethacin | 6 hours | 20 | 1 | n.d. |
| vehicle | 24 hours | 0 | 1 | n.d. |
| indomethacin | 24 hours | 2 | 1 | n.d. |
| indomethacin | 24 hours | 10 | 4.13 | n.d. |
| indomethacin | 24 hours | 20 | 8.41 | 13.68 |
| vehicle | 7days | 0 | 1 | n.d. |
| indomethacin | 7days | 2 | 1 | n.d. |
| indomethacin | 7days | 5 | 79.59 | 26.92 |

Table 1: Mean relative expression of rat FABP4 (SEQ. ID NO: 2) in the livers of WISTAR rats treated either once or for 7 consecutive days with different doses of indomethacin measured with Affymetrix microarray (RG U34A) or PCR (Applied Biosystems Assays on Demand ; assay ID: Rn00670361_ml). The expression is given relative to the expression in the respective time matched control animals treated with vehicle only. (n.d. = not determined)

## Claims

1. Use of FABP4 as biomarker for determining at least one toxic effect of a candidate compound.

2. The use according to claim 1, wherein the toxic effect is a hepatotoxic effect.

3. Method for predicting at least one toxic effect of a candidate compound comprising
a) detecting the level of expression of the FABP4 gene in a tissue or cell sample exposed to the compound
b) comparing the level of expression of the gene to its level of expression in a control tissue or cell sample, wherein a differential expression of the gene is indicative of at least one toxic effect.

4. Method according to claim 3, wherein a higher expression of the FABP4 gene in the tissue or cell sample exposed to the compound in comparison to the control is indicative for at least one toxic effect.

5. Method according to claim 3 or 4, wherein the at least one toxic effect is a hepatotoxic effect.


SEQUENCE LISTING

<110> F. Hoffmann-La Roche AG

<120> FABP4 as marker for a toxic effect

<130> 23334

<160> 3

<170> PatentIn version 3.3

<210> 1
<211> 619
<212> DNA
<213> Homo sapiens

<400> 1
tgcagcttcc ttctcacctt gaagaataat cctagaaaac tcacaaaatg tgtgatgctt 60

ttgtaggtac ctggaaactt gtctccagtg aaaactttga tgattatatg aaagaagtag 120

gagtgggctt tgccaccagg aaagtggctg gcatggccaa acctaacatg atcatcagtg 180

tgaatgggga tgtgatcacc attaaatctg aaagtacctt taaaaatact gagatttcct 240

tcatactggg ccaggaattt gacgaagtca ctgcagatga caggaaagtc aagagcacca 300

taaccttaga tgggggtgtc ctggtacatg tgcagaaatg ggatggaaaa tcaaccacca 360

taaagagaaa acgagaggat gataaactgg tggtggaatg cgtcatgaaa ggcgtcactt 420

ccacgagagt ttatgagaga gcataagcca agggacgttg acctggactg aagttcgcat 480

tgaactctac aacattctgt gggatatatt gttcaaaaag atattgttgt tttccctgat 540

ttagcaagca agtaattttc tcccaagctg attttattca atatggttac gttggttaaa 600

taactttttt tagatttag 619

<210> 2
<211> 600
<212> DNA
<213> Rattus norvegicus

<220>
<221> misc_feature
<222> (7)..(7)
<223> n is a, c, g, or t

<220>
<221> misc_feature
<222> (22)..(22)
<223> n is a, c, g, or t

<220>
<221> misc_feature
<222> (379)..(379)
<223> n is a, c, g, or t

<220>
<221> misc_feature
<222> (441)..(441)
<223> n is a, c, g, or t

<220>
<221> misc_feature
<222> (451)..(451)

<223>  n is a, c, g, or t

<220>
<221>  misc_feature
<222>  (525)..(525)
<223>  n is a, c, g, or t

<220>
<221>  misc_feature
<222>  (531)..(531)
<223>  n is a, c, g, or t

<220>
<221>  misc_feature
<222>  (545)..(545)
<223>  n is a, c, g, or t

<220>
<221>  misc_feature
<222>  (576)..(576)
<223>  n is a, c, g, or t

<400>  2
cttaggnacc ctgctagcag anaattcggc acgagtcctt gaaagcttac aaaatgtgcg      60

acgcctttgt ggggacctgg aaactcgtct ccagtgagaa cttcgatgat tacatgaaag     120

aagtgggagt tggcttcgcc accaggaaag tggccggtat ggccaagccc aacttgatca     180

tcagcgtaga aggggacttg gtcgtcatcc ggtcagagag tacttttaaa aacaccgaga     240

tttccttcaa actgggcgtg gaattcgatg aaatcacccc agatgacagg aaagtgaaga     300

gcatcataac cctggatggt ggggtcctgg tacatgtgca gaagtgggat ggaaagtcga     360

ccaccataaa gaggagacna gatggtgaca agctggtggt ggaatgtgtc atgaaaggcg     420

tgacttctac aagagtttac naaagggcat nagccaaggg acgaggccct ggactgaaat     480

ttgcatcaaa ctctacaata acttgatggg atttggtggt tttgntattt ntttttttaa     540

gatcnatttt gcactaatta gcaagcaatc actttnttct tgaactggga atttattgga     600

<210>  3
<211>  614
<212>  DNA
<213>  Mus musculus

<400>  3
cctttctcac ctggaagaca gctcctcctc gaaggtttac aaaatgtgtg atgcctttgt      60

gggaacctgg aagcttgtct ccagtgaaaa cttcgatgat tacatgaaag aagtgggagt     120

gggctttgcc acaaggaaag tggcaggcat ggccaagccc aacatgatca tcagcgtaaa     180

tggggatttg gtcaccatcc ggtcagagag tacttttaaa aacaccgaga tttccttcaa     240

actgggcgtg gaattcgatg aaatcaccgc agacgacagg aaggtgaaga gcatcataac     300

cctagatggc ggggccctgg tgcaggtgca gaagtgggat ggaaagtcga ccacaataaa     360

gagaaaacga gatggtgaca agctggtggt ggaatgtgtt atgaaaggcg tgacttccac     420

aagagtttat gaaagggcat gagccaaagg aagaggcctg gatggaaatt tgcatcaaac     480

actacaatag tcagtcggat ttattgtttt ttttaaagat atgattttcc actaataagc     540

aagcaattaa tttttctgaa agatgcattt tattggatat ggttatgttg attaaataaa     600

acctttttag actt 614